(19)

Europäisches
Patentamt
European
Patent Office
Office européen
des brevets

(11) **EP 1 839 575 A2**

(12) **EUROPEAN PATENT APPLICATION**

(43) Date of publication:
**03.10.2007 Bulletin 2007/40**

(51) Int Cl.:
*A61B 5/053* (2006.01)    *A61B 5/103* (2006.01)
*G06F 19/00* (2006.01)

(21) Application number: **07005509.0**

(22) Date of filing: **16.03.2007**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK YU**

(30) Priority: **29.03.2006 JP 2006090326**

(71) Applicant: **TANITA CORPORATION
Tokyo (JP)**

(72) Inventor: **Izumi, Miyuki
Tokyo (JP)**

(74) Representative: **Grünecker, Kinkeldey,
Stockmair & Schwanhäusser
Anwaltssozietät
Maximilianstrasse 58
80538 München (DE)**

(54)    **Health management apparatus**

(57)    Disclosed is a health management apparatus, which comprises age information acquisition means for acquiring age information of a user, gender information acquisition means for acquiring gender information of the user, body-height information acquisition means for acquiring body-height information of the user, body-weight information acquisition means for acquiring body-weight information of the user, bone-mass information acquisition means for acquiring bone-mass information of the user, and targeted amount of daily calcium intake per person information acquisition means for acquiring information about a targeted amount of daily calcium intake per person specific to the user, based on the acquired age information, gender information, body-height information, body-weight information and bone-mass information. The health management apparatus of the present invention can acquire a targeted amount of daily calcium intake per person specific to a user, in accordance with a body composition of the user.

FIG. 1

EP 1 839 575 A2

**Description**

BACKGROUND OF THE INVENTION

1. Field of the Invention

The present invention relates to a health management apparatus capable of acquiring information about a required calcium amount to be ingested by a user.

2. Description of the Related Art

Heretofore, there have been well known a technique and an apparatus designed to calculate a body fat mass or a body fat percentage of a user based on a body impedance value and others of the user, as disclosed, for example, in the following Patent Publications 1 and 2. Further, with a view to improving and advancing this apparatus to acquire information about body compositions of a user, such as a bone mass, a muscle mass and a basal metabolic rate, various technique and apparatuses have been proposed, as disclosed, for example, in the following Patent Publications 3, 4 and 5, and a part of these technique and apparatuses have already been put to practical use. In this specification, these conventional apparatuses as disclosed in the Patent Publications 1 to 5 will be referred to collectively as "body composition meter".

[Patent Publication 1] Japanese Patent Publication No. 05-049050
[Patent Publication 2] Japanese Patent No. 2835662
[Patent Publication 3] Japanese Patent Laid-Open Publication No. 2005-080976
[Patent Publication 4] Japanese Patent Laid-Open Publication No. 2004-344518
[Patent Publication 5] Japanese Patent Laid-Open Publication No. 2002-172099

SUMMARY OF THE INVENTION

The conventional body composition meter has no function of informing a user of information about a required calcium amount to be ingested by the user (hereinafter referred to as "targeted amount of daily calcium intake per person specific to the user" or "user-specific targeted amount of daily calcium intake per person"). On this point, it is contemplated to achieve the function in such a manner as to acquire a targeted amount of daily calcium intake per person specific to a user, using information about age, gender and body height of the user which are entered into the body composition meter as basic data for acquiring body composition information and in conformity to calcium-intake reference values recommend by the Ministry of Health, Labour and Welfare of Japan.

In reality, the calcium-intake reference values recommend by the Ministry of Health, Labour and Welfare of Japan are no more than data segmented by genders, and roughly-divided age groups and body-height classes. Thus, even if data about the calcium-intake reference values is simply incorporated in the conventional body composition meter, the function of acquiring a targeted amount of daily calcium intake per person specific to a user cannot be adequately obtained in accordance with a body composition of the user.

It is therefore an object of the present invention to provide a health management apparatus capable of acquiring a targeted amount of daily calcium intake per person specific to a user, in accordance with a body composition of the user.

In order to achieve the above object, the present invention provides a health management apparatus which comprises age information acquisition means for acquiring age information of a user, gender information acquisition means for acquiring gender information of the user, body-height information acquisition means for acquiring body-height information of the user, body-weight information acquisition means for acquiring body-weight information of the user, bone-mass information acquisition means for acquiring bone-mass information of the user, and targeted amount of daily calcium intake per person information acquisition means for acquiring information about a targeted amount of daily calcium intake per person specific to the user, based on the acquired age information, gender information, body-height information, body-weight information and bone-mass information.

In the health management apparatus of the present invention, the bone-mass information acquisition means preferably includes fat-free-mass information acquisition mean for acquiring fat-free mass information of the user, and bone-mass information calculation means for calculating the bone-mass information of the user, based on the acquired fat-free mass information.

Further, the fat-free-mass information acquisition mean preferably includes body-height information acquisition means for acquiring body-height information of the user, body-impedance information acquisition means for acquiring body-impedance information of the user, and fat-free-mass information calculation mean for calculating the fat-free mass information of the user, based on the acquired body-height information and body-impedance information.

Alternatively, the fat-free-mass information acquisition mean may include body-height information acquisition means for acquiring body-height information of the user, body-weight information acquisition means for acquiring body-weight information of the user, body-impedance information acquisition means for acquiring body-impedance information of the user, and fat-free-mass information calculation mean for calculating the fat-free mass information of the user, based on the acquired body-height information, body-weight information and body-impedance information.

Preferably, the health management apparatus of the present invention further includes muscle-mass information acquisition means for acquiring muscle-mass information of the user. In this case, the required-calcium-amount information acquisition means may be operable to acquire information about a targeted amount of daily calcium intake per person specific to the user, based on the acquired age information, gender information, body-height information, bone-mass information and muscle-mass information.

Further, the muscle-mass information acquisition means preferably includes bone-mass information acquisition means for acquiring bone-mass information of the user, fat-free-mass information acquisition mean for acquiring fat-free mass information of the user, and muscle-mass information calculation means for calculating the muscle-mass information of the user, based on the acquired bone-mass information and fat-free mass information.

Preferably, the health management apparatus of the present invention further includes a storage device which stores conversion data corresponding to targeted amount of daily calcium intake per person information, and a screen adapted to display the conversion data corresponding to the acquired information about the targeted amount of daily calcium intake per person specific to the user.

As above, the health management apparatus of the present invention comprises means for acquiring age information, gender information, body-height information, body-weight information and bone-mass information of a user, and means for acquiring information about a targeted amount of daily calcium intake per person specific to the user, based on the acquired age information, gender information, body-height information, body-weight information and bone-mass information. Thus, the user-specific targeted amount of daily calcium intake per person information is acquired based on not only the age information, gender information and body-height information of the user but also the information about bone mass having a close relation to calcium. That is, the health management apparatus of the present invention makes it possible to acquire information about a targeted amount of daily calcium intake per person specific to a user, in accordance with a bone mass as a body composition of the user.

In the present invention, the means for acquiring bone-mass information may comprise means for acquiring fat-free mass information, and means for calculating bone-mass information based on the acquired fat-free mass information. This makes it possible to provide a highly-reliable health management apparatus based on an existing technique.

Further, the means for acquiring fat-free mass information may comprise means for acquiring body-height information, means for acquiring body-impedance information, and mean for calculating fat-free mass information, based on the acquired body-height information and body-impedance information, or may comprise means for acquiring body-height information, means for acquiring body-weight information, means for acquiring body-impedance information, and mean for calculating fat-free mass information based on the acquired body-height information, body-weight information and body-impedance information. This makes it possible to provide a highly-reliable health management apparatus in a simplified manner, i.e., at a lower cost, based on an existing technique and apparatus.

The health management apparatus of the present invention may further include means for acquiring muscle-mass information. In this case, the user-specific targeted amount of daily calcium intake per person information is acquired based on not only the age information, gender information and body-height information of the user but also the information about bone mass having a close relation to calcium, and the information about muscle mass. That is, this health management apparatus makes it possible to acquire information about a targeted amount of daily calcium intake per person specific to a user, in accordance with a bone mass and a muscle mass as body compositions of the user.

In this health management apparatus, the means for acquiring muscle-mass information may comprise means for acquiring bone-mass information, mean for acquiring fat-free mass information, and means for calculating muscle-mass information based on the acquired bone-mass information and fat-free mass information. This makes it possible to provide a highly-reliable health management apparatus based on an existing technique. Further, the means for acquiring bone-mass information and the mean for acquiring fat-free mass information may comprise the means for calculating bone-mass information based on the acquired fat-free mass information, the means for acquiring body-height information, the means for acquiring body-impedance information, and the mean for calculating fat-free mass information based on the acquired body-height information and body-impedance information. This makes it possible to provide a highly-reliable health management apparatus in a simplified manner, i.e., at a lower cost, based on an existing technique and apparatus.

BRIEF DESCRIPTION OF THE DRAWINGS

FIG 1 is an external view showing a health management apparatus 1 according to one embodiment of the present invention.

FIG 2 is a block diagram showing a schematic configuration of an electric circuit incorporated in the health management apparatus 1.

FIG. 3 is a flowchart showing a personal data registration process to be performed in the health management apparatus 1.

FIG 4 is a flowchart showing a biological data measurement process to be performed in the health management apparatus 1.

FIGS. 5A and 5B are charts showing one example of a data table to be stored in the health management apparatus 1.

FIGS. 6A and 6B are charts showing another example of the data table to be stored in the health management apparatus 1.

FIG 7 is a chart showing yet another example of the data table to be stored in the health management apparatus 1.

FIG 8 is a chart showing still another example of the data table to be stored in the health management apparatus 1.

FIG 9 is a diagram showing one example of a biological data display mode to be performed in the health management apparatus 1.

## THE PREFERRED EMBODIMENTS OF THE INVENTION

A health management apparatus of the present invention comprises age information acquisition means for acquiring age information of a user, gender information acquisition means for acquiring gender information of the user, body-height information acquisition means for acquiring body-height information of the user, body-weight information acquisition means for acquiring body-weight information of the user, bone-mass information acquisition means for acquiring bone-mass information of the user, and targeted amount of daily calcium intake per person information acquisition means for acquiring information about a targeted amount of daily calcium intake per person specific to the user, based on the acquired age information, gender information, body-height information, body-weight information and bone-mass information.

In the health management apparatus of the present invention, the bone-mass information acquisition means preferably includes fat-free-mass information acquisition mean for acquiring fat-free mass information of the user, and bone-mass information calculation means for calculating the bone-mass information of the user, based on the acquired fat-free mass information.

Further, the fat-free-mass information acquisition mean preferably includes body-height information acquisition means for acquiring body-height information of the user, body-impedance information acquisition means for acquiring body-impedance information of the user, and fat-free-mass information calculation mean for calculating the fat-free mass information of the user, based on the acquired body-height information and body-impedance information.

Alternatively, the fat-free-mass information acquisition mean may include body-height information acquisition means for acquiring body-height information of the user, body-weight information acquisition means for acquiring body-weight information of the user, body-impedance information acquisition means for acquiring body-impedance information of the user, and fat-free-mass information calculation mean for calculating the fat-free mass information of the user, based on the acquired body-height information, body-weight information and body-impedance information.

Preferably, the health management apparatus of the present invention further includes muscle-mass information acquisition means for acquiring muscle-mass information of the user. In this case, the required-calcium-amount information acquisition means may be operable to acquire information about a targeted amount of daily calcium intake per person specific to the user, based on the acquired age information, gender information, body-height information, bone-mass information and muscle-mass information.

Further, the muscle-mass information acquisition means preferably includes bone-mass information acquisition means for acquiring bone-mass information of the user, fat-free-mass information acquisition mean for acquiring fat-free mass information of the user, and muscle-mass information calculation means for calculating the muscle-mass information of the user, based on the acquired bone-mass information and fat-free mass information.

Preferably, the health management apparatus of the present invention further includes a storage device which stores conversion data corresponding to targeted amount of daily calcium intake per person information, and a screen adapted to display the conversion data corresponding to the acquired information about the targeted amount of daily calcium intake per person specific to the user.

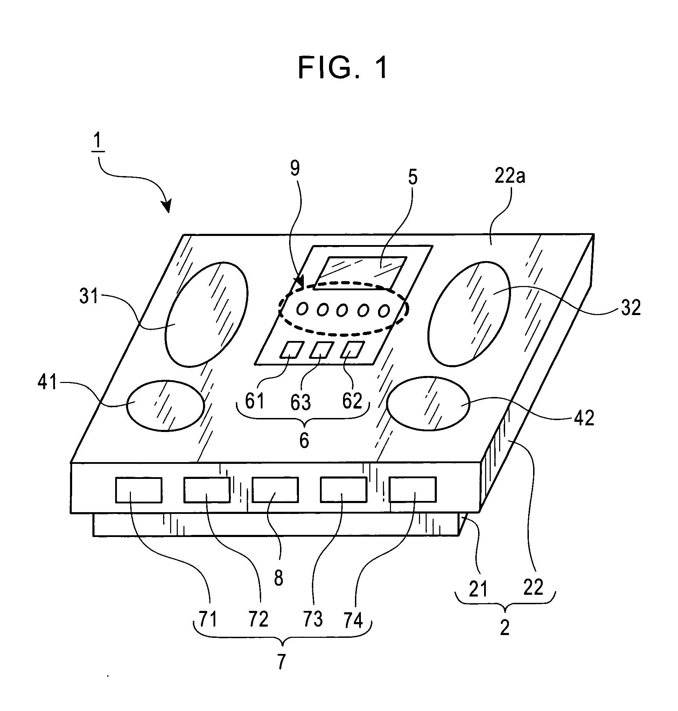

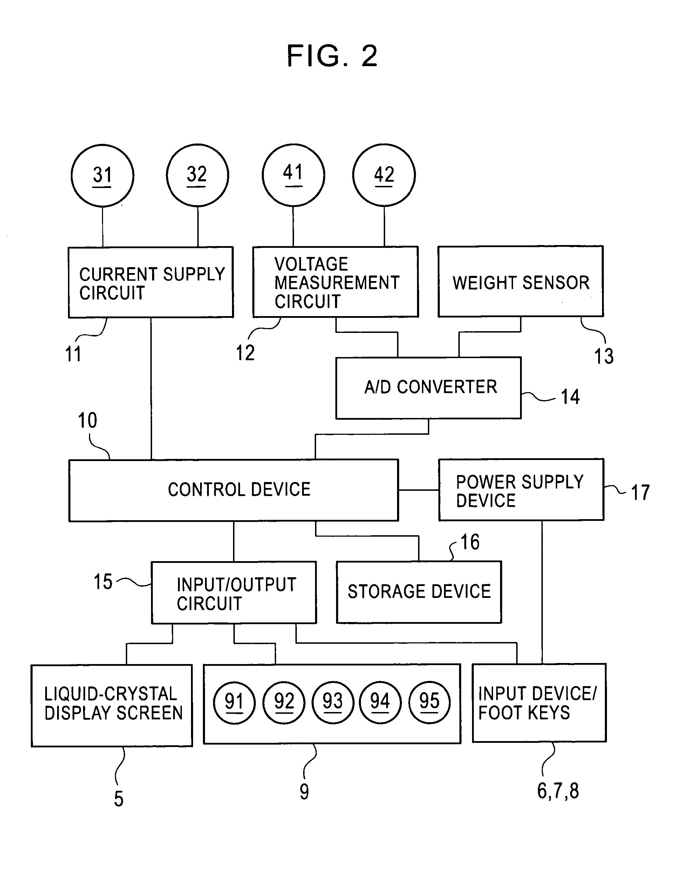

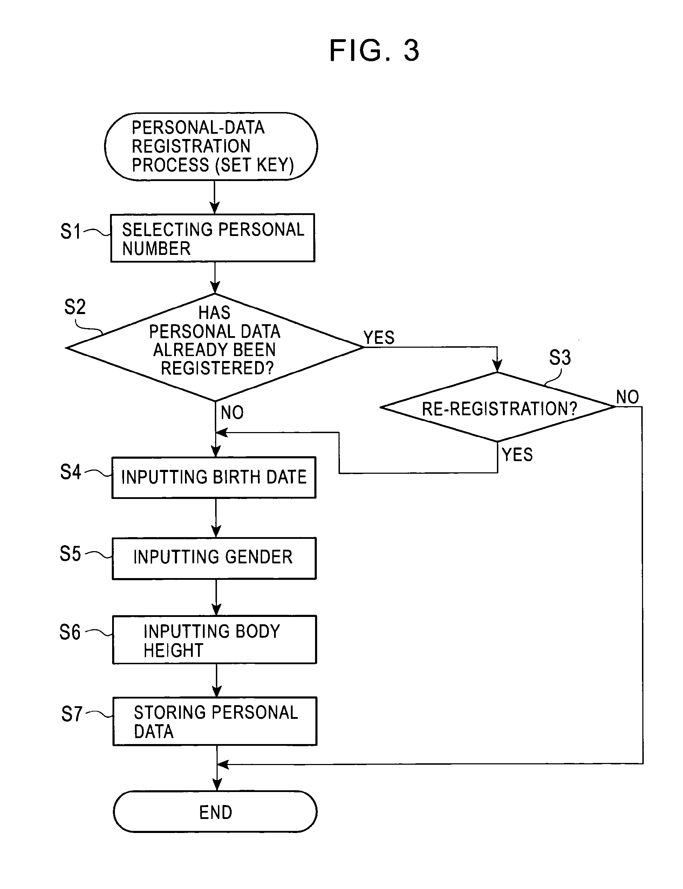

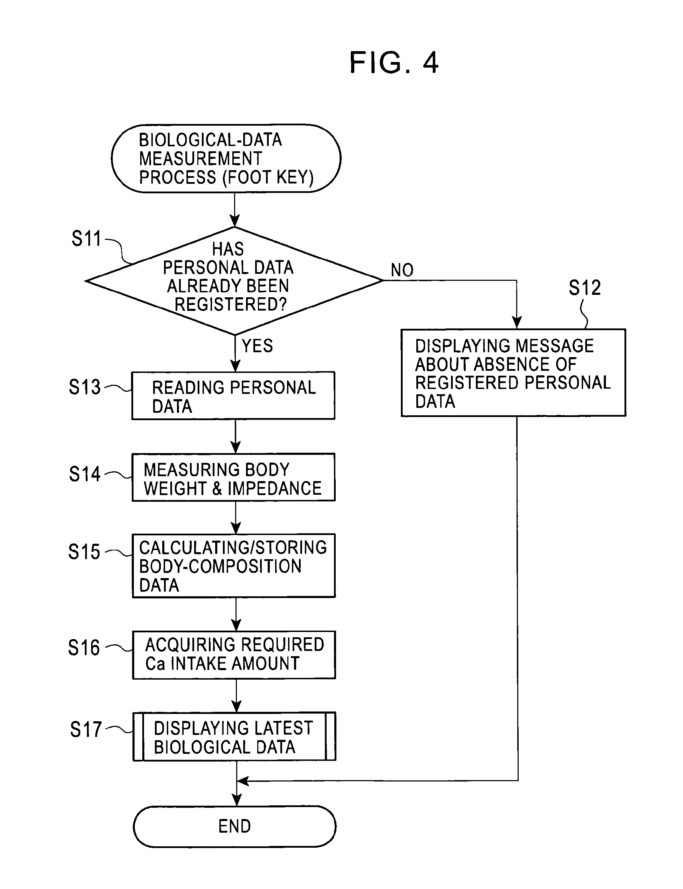

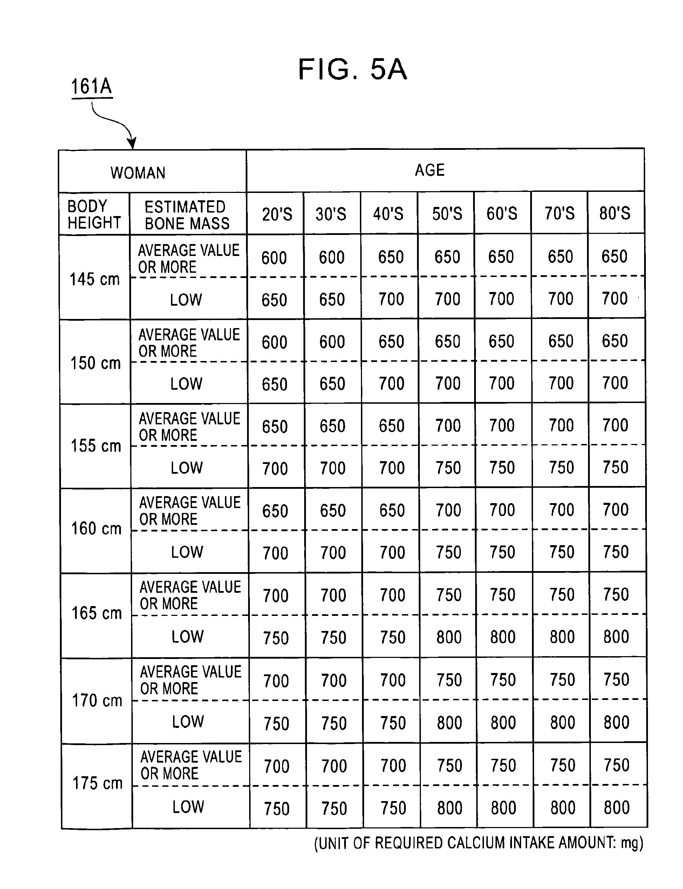

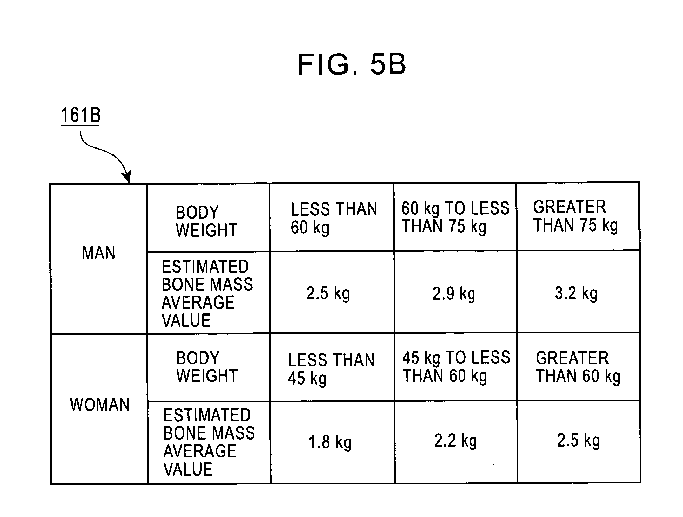

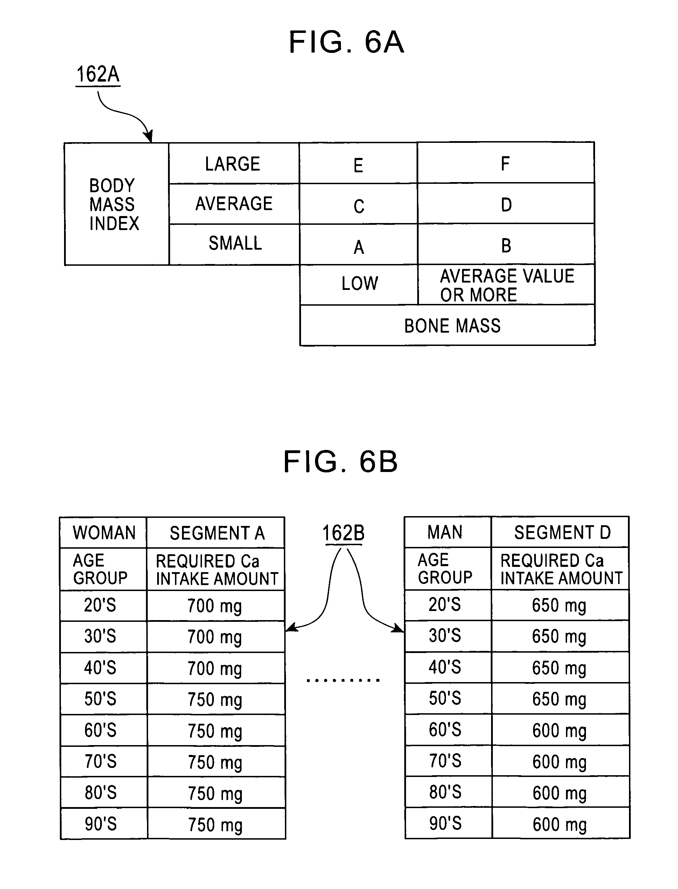

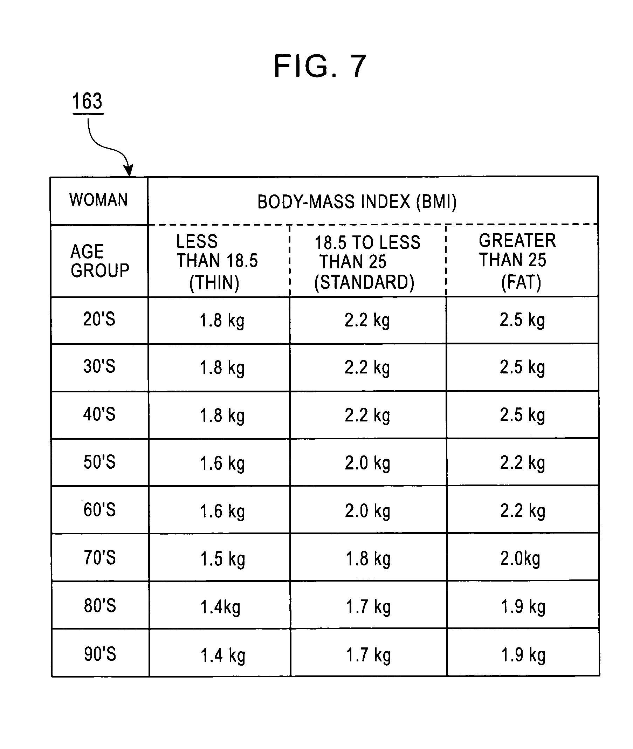

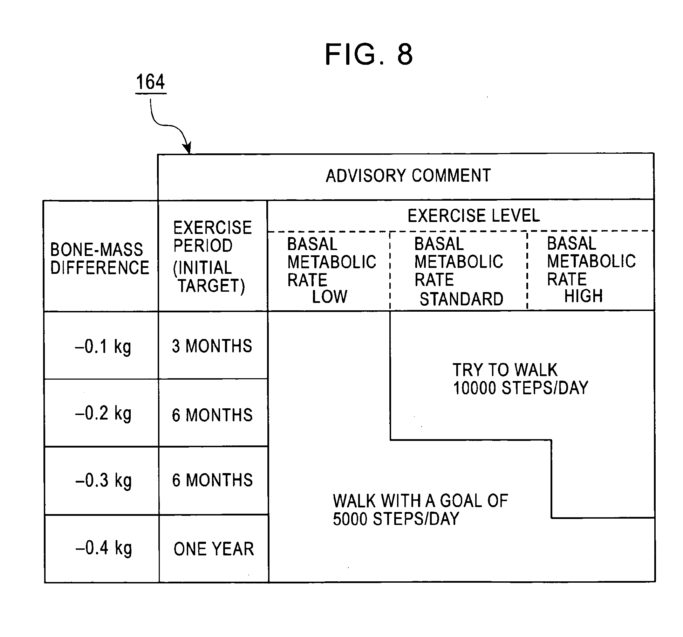

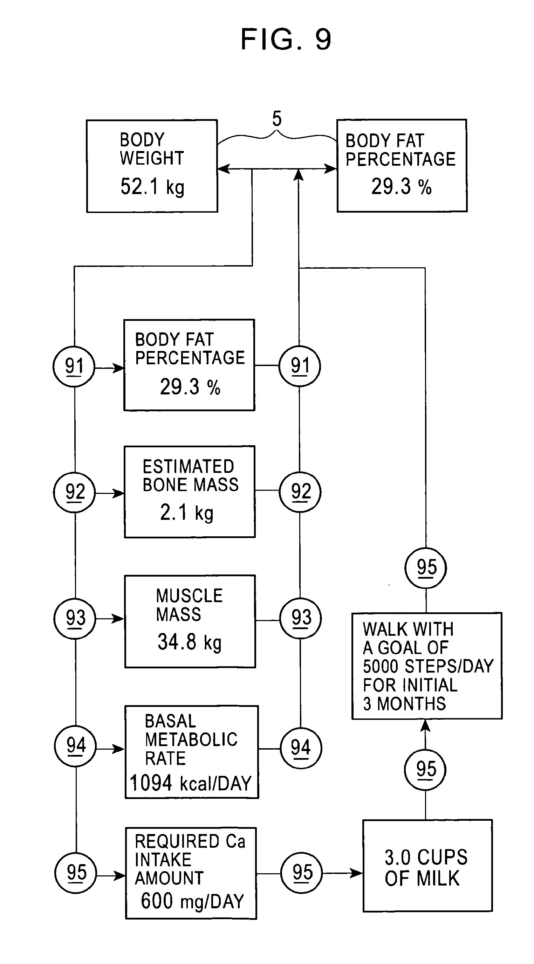

With reference to the drawings, a preferred embodiment of the present invention will be described. FIG 1 is an external view showing a health management apparatus 1 according to one embodiment of the present invention. FIG 2 is a block diagram showing a schematic configuration of an electric circuit incorporated in the health management apparatus 1. FIG 3 is a flowchart showing a personal data registration process to be performed in the health management apparatus 1. FIG 4 is a flowchart showing a biological data measurement process to be performed in the health management apparatus 1. FIGS. 5A and 5B are charts showing one example of a data table to be stored in the health management apparatus 1. FIGS. 6A and 6B are charts showing another example of the data table to be stored in the health management apparatus 1. FIG 7 is a chart showing yet another example of the data table to be stored in the health management apparatus 1. FIG 8 is a chart showing yet another example of the data table to be stored in the health management apparatus 1. FIG 9 is a diagram showing one example of a biological data display mode to be performed in the health management apparatus 1.

This health management apparatus 1 (hereinafter referred to shortly as "apparatus 1") is intended to improve a conventional body composition meter designed to calculate body-composition data of a user, such as a body fat percentage, a bone mass, a muscle mass and a basal metabolic rate, based on age data, gender data, body-height data, body-weight data and inter-foot body-impedance data of the user, in such a manner as to allow a user-specific targeted amount of daily calcium intake per person to be additionally calculated based on some of the above data. In this specification, the age data, the gender data and the body-height data will be occasionally referred to collectively as "personal data". Further, the total six data consisting of the body-weight data, the four types of body-composition data and the user-specific targeted amount of daily calcium intake per person will be occasionally referred to collectively as "biological data".

As shown in FIG 1, the apparatus 1 has a main body 2 comprising a base 21 and a load-receiving platform 22 mounted on the base 21. The platform 22 has a top surface 22a provided with a pair of current supply electrodes 31, 32 for supplying an AC current between the right and left feet bottoms of a user, a pair of measurement electrodes 41, 42 for measuring a voltage (potential difference) to be generated between the right and left feet bottoms in response to the current supply, a liquid-crystal display screen 5 for displaying personal data and biological data, and an input device 6 for allowing the user to input personal data therethrough.

The input device 6 includes an up key 61, a down key 62 and a set key 63 which are used for manual operations of selecting and setting data to be displayed on the liquid-crystal display screen 5. The main body 2 has a side surface provided with a foot key group 7 which consists of four foot keys 71, 72, 73, 74 for calling up registered personal data if any, and a foot key 8 for turning off power. Each of the set key 63 and the foot keys 71 to 74 additionally serves as a power-on key. Further, as indicated by the dashed circle in FIG 1, an item key group 9 which consists of five key switches 91, 92, 93, 94 and 95 is arranged on the top surface 22a of the platform 22 at a position between the liquid-crystal display screen 5 and the input device 6. These five key switches 91 to 95 are associated with biological data except for body-weight data (i.e., body fat percentage, bone mass, muscle mass, basal metabolic rate and user-specific targeted amount of daily calcium intake per person), respectively.

As shown in FIG 2, the apparatus 1 internally comprises: a current supply circuit 11 electrically connected to the current supply electrodes 31, 32; a voltage measurement circuit 12 electrically connected to the measurement electrodes 41, 42; a weight sensor 13 adapted to output a voltage corresponding to a load imposed thereon so as to measure body-weight data of a user; an A/D converter 14 for converting a voltage signal from each of the voltage measurement circuit 12 and the weight sensor 13 to a digital signal; an input/output circuit 15 electrically connected to each of the liquid-crystal display screen 5, the input device 6, the foot key group 7, the power-off key 8 and the item key group 9 (key switches 91 to 95); a storage device 16 for storing the entered personal data and the measured biological data; a power supply device 17 including a battery; and a control device 10 electrically connected to each of the current supply circuit 11, the A/D converter 14, the input/output circuit 15, the storage device 16 and the power supply device 17.

In the above configuration, the control device 10 includes a conventional computing element (CPU). The control device 10 is operable to constantly update current date/hour according to a built-in clock and execute a control program pre-stored in the storage device 16 so as to perform various control processes, such as a process of accepting an input of personal data from the input device 6; a process of measuring body-weight data using the weight sensor 13; a process of supplying an AC current to the current supply electrodes 31, 32; a process of calculating a body impedance of a user based on the supplied current value and a voltage value detected by the measurement electrodes 41, 42; a process of calculating body-composition data and a user-specific targeted amount of daily calcium intake per person based on the calculated body impedance, the personal data entered from the input device 4 and the body-weight data measured by the weight sensor 13; a process of displaying the entered personal data and the measured biological data on the liquid-crystal display screen 5 and storing these data in the storage device 16, to measure biological data of the user. With reference to FIGS. 3 to 6, these control processes will be described below.

The flowchart in FIG 3 shows a process to be performed when a user, for example, who uses the apparatus 1 for the first time, registers his/her personal data. This registration process is initiated when a user presses down the set key 63 to activate the apparatus 1.

In Step S1, a personal number of the user is set up. Specifically, four numerical characters 1 to 4 serving as optional personal numbers are displayed on the liquid-crystal display screen 5. The user manipulates the up key 61 and/or the down key 62 to select any one of the displayed numerical characters as his/her personal number, and then presses down the set key 63 to set the selected numerical character as his/her personal number. Then, in Step S2, it is determined whether personal data associated with the selected personal number has already been registered. When the personal data has already been reregistered, the process advances to Step S3. If no personal data has been reregistered, the process will advance to Step S4. In Step S3, options inquiring about whether the user requests for performing a re-registration are displayed on the liquid-crystal display screen 5. If the user selects one option indicative of requesting for the re-registration, the process will advance to Step S4. When the user selects the other option indicative of skipping the re-registration, this registration process is terminated, and the power supply is automatically turned off.

In Step S4, the birth date of the user is entered. Specifically, numerical characters representing year, month and day are displayed on the liquid-crystal display screen 5. The user manipulates the up key 61 and/or the down key 62 to increase and/or reduce each of the numerical characters so as to adjust the numerical characters to his/her birth date, and then presses down the set key 63 to set the adjusted numerical characters as his/her birth date. The control device 10 is operable to compare the entered birth date with a current date indicated by the built-in clock so as to calculate age data of the user. Then, in Step S5, gender data of the user is entered. Specifically, options about gender are displayed on the liquid-crystal display screen 5. The user manipulates the up key 61 and/or the down key 62 to select his/her gender, and then pressed down the set key 63 to set the selected option as his/her gender. Then, in Step S6, body-height data of the user is entered. Specifically, numerical characters representing body height are displayed on the liquid-crystal display screen 5. The user manipulates the up key 61 and/or the down key 62 to increase and/or reduce each of the numerical characters so as to adjust the numerical characters to his/her body height, and then presses down the set key 63 to set the selected numerical characters as his/her body height.

In Step S7, the personal number selected in Step S1 and the personal data entered in Steps S4 to S6 are stored in the storage device 16 in association with each other. Through the above steps, the personal-data registration process is fully completed. Then, after an elapse of a given time (e.g., about 10 seconds), the power supply will be automatically turned off. During this time period, the entered personal data may be displayed on the liquid-crystal display screen 5 to allow the user to confirm them. It is understood that the user may press down the power-off key 8 to turn off power.

The flowchart in FIG 4 shows a process to be performed when the user measures his/her biological data using the apparatus 1. This measurement process is initiated when the user presses down one of the foot key group 7 (one of the foot key 71 to 74) which corresponds to the personal number associated with the personal data of the user to activate the apparatus 1.

In Step S11, it is determined whether personal data corresponding to the pressed foot key 7 has already been registered. If no personal data has been registered, the process will advance to Step S12 to prompt the user to register his/her personal data. In Step S12, a message indicative of the absence of registered personal data is displayed on the liquid-crystal display screen 5 for a given time, and then this measurement process is terminated. Instead of terminating the measurement process, the process may be shifted to Step S4 in FIG 4. When the personal data has already been registered, the process advances to Step S 13 to read the personal data, and then process advances to Step 14.

In Step S14, body-weight data and inter-foot body-impedance data of the user are measured. Specifically, when the user stands on the top surface 22a of the platform 22 in such a manner that the left foot bottom comes into contact with the current supply electrode 31 and the measurement electrode 41, and the right foot bottom comes into contact with the current supply electrode 32 and the measurement electrode 42, a load detected by the weight sensor 13 is measured as body-weight data of the user. Simultaneously, an AC current is supplied between the right/left feet bottoms through the current electrodes 31, 32, and a voltage (potential difference) between the right/left feet bottoms is measured through the measurement electrodes 41, 42. Then, an inter-foot bioelectric impedance is calculated based on the supplied current value and the measured voltage value. Alternatively, a plurality of reference resistors each having a different known resistance value may be inserted into the electric circuit in series or parallel to the user's body, and potential differences across the respective resistors may be measured together with a potential difference generated between the user's feet, to calculate a user's body impedance based on a ratio between each of the measured potential differences and each of the resistance values of the corresponding reference resistors. In this case, the body-impedance data can be obtained even if a value of current to be supplied to the user's body is unknown.

Then, in Step S15, body-fat-percentage data, bone-mass data, muscle-mass data and basal- metabolic-rate data are calculated based on the age data, gender data and body-height data which have been registered as the personal data of the user, and the body-weight data and the body-impedance data which have been measured in Step S14. Then, these calculated data are stored in the storage device 16. A method of calculating these data will be described below.

A body density BD of the user is firstly calculated based on the body-height data Ht, the body-weight data Wt and the body-impedance data Z and using the following Formula 1, and then a body fat percentage %FAT is calculated based on the calculated body density BD and using the following Formula 2:

$$BD = a - b \times Wt \times Z / Ht^2 \qquad \text{(Formula 1)}$$

$$\% \text{FAT} = (4.95 \,/\, \text{BD} - 4.5) \times 100 \qquad \text{(Formula 2)}$$

In Formula 1, each of "a" and "b" is a predetermined constant for each of the gender data. This calculation method is specifically disclosed in the aforementioned Patent Publications 1 and 2.

Then, a fat-free mass FFM of the user is calculated based on the body-height data Ht and the body-impedance data Z and using the following Formula 3, and then a bone mass BMC is calculated based on the calculated fat-free mass FFM and using the following Formula 4:

$$\text{FFM} = \text{Ht}^2 \times \text{K} \,/\, \text{Z} \qquad \text{(Formula 3)}$$

$$\text{BMC} = \text{C1} \times \text{FFM} + \text{C2} \qquad \text{(Formula 4)}$$

In Formula 3, K is a constant pre-determined on the basis of a volume resistivity of fat-free tissue and a specific gravity of fat-free tissue. In Formula 4, each of C1 and C2 is a predetermined constant for each of the gender data. This calculation method is specifically disclosed in the aforementioned Patent Publication 3.

Alternatively, the fat-free mass FFM may be calculated based on the body-weight data Wt and the body-impedance data Z and using the following Formula 5 :

$$\text{FFM} = \text{Wt} \times (1 - \%\text{FAT}) \,/\, 100 \qquad \text{(Formula 5)}$$

Then, a muscle mass MM of the user is calculated based on the fat-free mass FFM and the bone mass BMC and using the following Formula 6:

$$\text{MM} = \text{FFM} - \text{BMC} \qquad \text{(Formula 6)}$$

This calculation method is specifically disclosed in the aforementioned Patent Publication 4.

Then, a basal metabolic rate BMR of the user is calculated based on the age data Age and the fat-free mass FFM and using the following Formula 7:

$$\text{BMR} = \text{A} \times \text{FFM} + \text{B} \times (1 \,/\, \text{Age}) + \text{C} \qquad \text{(Formula 7)}$$

In Formula 7, each of A, B and C is a predetermined constant for each of the gender data. This calculation method is specifically disclosed in the aforementioned Patent Publication 5.

Then, in Step S16, information about a required calcium amount to be ingested by the user, i.e., a targeted amount of daily calcium intake per person specific to the user, is acquired based on the age data, the gender data, the body-height data, the body-weight data and the bone-mass data. Specifically, the storage device 16 stores a data table 161 A representing targeted amount of daily calcium intake per persons, as shown in FIG 5A, and a data table 161B representing estimated bone-mass average values segmented by genders and body-weight classes, as shown in FIG 5B, and the control device 10 is operable to read one of the targeted amount of daily calcium intake per persons which corresponds to the respective data acquired in Steps S13 to 15.

In the data table 161A, "average value or more" in the estimated bone mass is selected when the bone mass calculated in Step 15 is equal to or greater than 2.2 Kg, and "low" in the estimated bone mass is selected when the bone mass calculated in Step 15 is less than 2.2 Kg. This threshold value "2.2 Kg" is determined in accordance with the estimated bone-mass average values segmented by genders and body-weight classes in the data table 161B which has been obtained as the result of long years of research made by the applicant of this application. For example, when the user is a woman in 20's who has a body height of 155 cm, a body weight of 45 kg and a bone mass of 2.1 kg, a targeted amount of daily calcium intake per person specific to the user is 700 mg.

Instead of the data table 161A, a data table 162A as shown in FIG 6A and a data table 162B as shown in FIG 6B may be stored in the storage device 16. Specifically, the data table 162A is designed to allow users to be segmented by a parameter consisting of body-mass-index (BMI) data and bone-mass data, and the data table 162B is designed to allow a user-specific targeted amount of daily calcium intake per person to be determined by a parameter consisting of the user segment data in the data table 162A, the gender data and the age data. The body-mass index means a value obtained by dividing a body weight by a square value of a body height. In the data table 162A, "average value or more" in the bone mass is selected when the user is a woman having a body weight of 45 kg to less than 60 kg, and the bone mass calculated in Step 15 is 2.2 kg or more. Further, "low" in the bone mass is selected when the user is a woman having a body weight of 45 kg to less than 60 kg, and the bone mass calculated in Step 15 is less than 2.2 kg. This threshold value "2.2 Kg" is determined in accordance with the estimated bone-mass average values segmented by genders and body-weight classes in the data table 161B (FIG 5A). In this case, the control device 10 is operable to calculate body-mass-index data based on the body-height data and the body-weight data acquired in Steps S 13 and S 14. Then, the control device 10 is operable to read, from the data table 162A, the user segment data corresponding to the calculated body-mass-index data and the bone-mass data acquired in Step S 15, and then read, from the data table 162B, the targeted amount of daily calcium intake per person corresponding to the read user segment data, and the age data and the gender data acquired in Step 13 as a user-specific targeted amount of daily calcium intake per person.

Instead of the data table 162A using a body mass index, a data table (not shown) designed to allow users to be segmented by a parameter consisting of muscle-index (LMI) data and bone-mass data. The muscle index means a value obtained by dividing a muscle mass by a square value of a body height. In this case, the control device 10 is operable to calculate muscle-index data based on the body-height data and the muscle-mass data acquired in Steps S13 to S15. Then, the control device 10 is operable to read, from the above alternative data table (not shown), the user segment data corresponding to the calculated muscle-index data and the bone-mass data acquired in Step S 15, and then read, from the data table 162B, the targeted amount of daily calcium intake per person corresponding to the read user segment data, and the age data and the gender data acquired in Step 13 as a user-specific targeted amount of daily calcium intake per person.

As above, instead of the calcium-intake reference values recommend by the Ministry of Health, Labour and Welfare of Japan which are no more than data segmented by genders, and roughly-divided age groups and body-height classes, the health management apparatus 1 according to this embodiment employs the data table 161 (161A and 161B) to allow information about a user-specific targeted amount of daily calcium intake per person to be additionally acquired based on a bone mass having a close relation to calcium. Further, the data tables 162A and 162A used in place of the data table 161A, or the data table using a muscle index instead of the body-mass index as one of the parameters of the data table 162A, makes it possible to acquire information about a user-specific targeted amount of daily calcium intake per person, based on not only a bone mass but also a body weight and a muscle mass

In Step S16, the following process is additionally performed to provide a useful advice for a user's health management, in association with the user-specific targeted amount of daily calcium intake per person read from the data table 161.

The storage device 16 stores a conversion data table (not shown) for converting a user-specific targeted amount of daily calcium intake per person to easily-understandable data, such as "n cups of milk", and the control device 16 is operable to read, from the conversion data table, conversion data corresponding to the user-specific targeted amount of daily calcium intake per person read from the data table 161. Instead of "n cups of milk, this conversion data may have any other suitable form, such as "n bottles of milk", "n grams of cheese" or "n grams of little fishes".

The storage device 16 further stores a data table 163 representing required bone masses segmented by a parameter consisting of three types of data, i.e., the age data, the gender data and the body-mass-index (BMI) data, as show in FIG 7, and the control device 10 is operable to read the required bone-mass data from the data table 163, based on the age data, the gender data, the body-height data and the body-weight data of the user which have been acquired in Steps S13 to S15. The required bone mass means a minimum required bone mass for maintaining a current physique of a user. The required-bone-mass data can be pre-determined and segmented by genders, ages (age groups) and body-mass indexes. The body-mass index is a known index obtained by dividing a body weight by a square value of a body height, as mentioned above.

The control device 10 is operable to calculate bone-mass-difference data which is a difference between the bone-mass data calculated in Step S 15 and the required-bone-mass data read from the data table 163. For example, when the calculated bone-mass data is 2.1 kg, and the read required-bone-mass data is 2.2 kg, the bone-mass-difference data is - 0.1 kg.

The storage device 16 further stores a data table 164 representing advisory comments segmented by a parameter consisting of bone-mass-difference data and basal-metabolic-rate data, as shown in FIG 8, and the control device 10 is operable to read the advisory comment from the data table 164, based on the basal-metabolic-rate data calculated in Step S 15 and the above bone-mass-difference data calculated in Step 16. For example, when the calculated basal-metabolic-rate data is lower than a given level (fat is not efficiently burnt), and the calculated bone-mass-difference data is - 0.1 kg, an advisory comment "Walk with a goal of 5000 steps/day for initial 3 months" will be read. The advisory comment is not limited to the example as shown in FIG 8, but may be any other suitable form, such as "In view of increasing the bone mass, it is essential to increase the body weight and build additional muscle. Let's start with consuming an adequate diet. Calcium can be ingested from the diet. It is also good for bones.", "Bones and muscles have close ties with each other. If the muscles decrease, the bone mass will gradually decrease. Turn your attention to muscles and bones without being content to just manage the body weight.", or "Take care not to gain any further weight. It will impose a load on the bones. Reform your diet, for example, in such a manner as to mainly ingest vegetables and fishes. Try to walk at least 5000 steps/day, and gradually increase walking steps."

After completion of Step S16, in Step 17, the body-weight data which has been measured in Step S 14, and the body-fat-percentage data, the bone-mass data, the muscle-mass data and the basal-metabolic-rate data which have been calculated in Step 15, are displayed on the liquid-crystal display screen 5.

Specifically, as shown in FIG 9, the body-weight data and the body-fat-percentage data are alternately displayed on the liquid-crystal display screen 5 at time intervals of about 3 seconds, 6 times in total (i.e., each of the two data is displayed 3 times). If one of the item key group 9 is pressed down during the alternate display mode, one of the body-fat-percentage data, the bone-mass data, the muscle-mass data, the basal-metabolic-rate data and the user-specific targeted amount of daily calcium intake per person data will be displayed in response to the pressed one of the key switches 91 to 95. That is, the body-fat-percentage data will be displayed in response to pressing down the key switch 91 corresponding to body fat percentage, and the bone-mass data will be displayed in response to pressing down the key switch 92 corresponding to bone mass. Further, the muscle-mass data will be displayed in response to pressing down the key switch 93 corresponding to muscle mass, and the basis-metabolic-rate data will be displayed in response to pressing down the key switch 94 corresponding to basis metabolic rate. Furthermore, the user-specific targeted amount of daily calcium intake per person data will be displayed in response to pressing down the key switch 94 corresponding to user-specific targeted amount of daily calcium intake per person. In this state, if a different one of the key switches is pressed down, the current display will be switched to display one of the five data which corresponds to the newly-pressed key switch. Further, when the same key switch is re-pressed down, the liquid-crystal display screen 5 will be returned to the mode of alternately displaying the body-weight data and the body-fat-percentage data. Exceptionally, the user-specific targeted amount of daily calcium intake per person data is additionally displayed in the following display mode.

If the key switch 95 is pressed down when the user-specific targeted amount of daily calcium intake per person data is being displayed, the conversion data indicating "n cups of milk" acquired in Step 16 will be displayed in place of the currently-displayed user-specific targeted amount of daily calcium intake per person data. In this state, if the key switch 95 is re-pressed down, the advisory comment acquired in Step 16 will be displayed. In this state, if the key switch 95 is re-pressed down, the liquid-crystal display screen 5 will be returned to the mode of alternately displaying the body-weight data and the body-fat-percentage data.

After completion of the mode of alternately displaying the body-weight data and the body-fat-percentage data 6 times in total, or when the foot key 8 for turning off power is pressed down, the measurement mode is terminated, and the power supply is turned off.

As mentioned above, the health management apparatus 1 according to one embodiment of the present invention can acquire information about a targeted amount of daily calcium intake per person specific to a user, in accordance with a body composition of the user, particularly, a bone mass having a close relation to calcium. While the present invention has been described based on the specific embodiment, it is understood that the present invention is not limited to the embodiment. In addition to the modifications described in connection with the embodiment, various other changes and modifications may be made therein without departing from the spirit and scope of the invention as set forth in the appended claims.

## Claims

1. A health management apparatus comprising:

   age information acquisition means for acquiring age information of a user;
   gender information acquisition means for acquiring gender information of the user;
   body-height information acquisition means for acquiring body-height information of the user;
   body-weight information acquisition means for acquiring body-weight information of the user;
   bone-mass information acquisition means for acquiring bone-mass information of the user; and
   targeted amount of daily calcium intake per person information acquisition means for acquiring information about a targeted amount of daily calcium intake per person specific to the user, based on said acquired age information, gender information, body-height information, body-weight information and bone-mass information.

2. The health management apparatus as defined in claim 1, wherein said bone-mass information acquisition means includes:

fat-free-mass information acquisition mean for acquiring fat-free mass information of the user; and
bone-mass information calculation means for calculating the bone-mass information of the user, based on said acquired fat-free mass information.

3. The health management apparatus as defined in claim 2, wherein said fat-free-mass information acquisition mean includes:

body-height information acquisition means for acquiring body-height information of the user;
body-impedance information acquisition means for acquiring body-impedance information of the user; and
fat-free-mass information calculation mean for calculating the fat-free mass information of the user, based on said acquired body-height information and body-impedance information.

4. The health management apparatus as defined in claim 2, wherein said fat-free-mass information acquisition mean includes:

body-height information acquisition means for acquiring body-height information of the user;
body-weight information acquisition means for acquiring body-weight information of the user;
body-impedance information acquisition means for acquiring body-impedance information of the user; and
fat-free-mass information calculation mean for calculating the fat-free mass information of the user, based on said acquired body-height information, body-weight information and body-impedance information.

5. The health management apparatus as defined in either one of claims 1 to 4, which further includes muscle-mass information acquisition means for acquiring muscle-mass information of the user, wherein said required-calcium-amount information acquisition means is operable to acquire information about a targeted amount of daily calcium intake per person specific to the user, based on said acquired age information, gender information, body-height information, bone-mass information and muscle-mass information.

6. The health management apparatus as defined in claim 5, wherein said muscle-mass information acquisition means includes:

bone-mass information acquisition means for acquiring bone-mass information of the user; fat-free-mass information acquisition mean for acquiring fat-free mass information of the user; and
muscle-mass information calculation means for calculating the muscle-mass information of the user, based on said acquired bone-mass information and fat-free mass information.

7. The health management apparatus as defined in claim 1, which further includes:

a storage device which stores conversion data corresponding to targeted amount of daily calcium intake per person information; and
a screen adapted to display the conversion data corresponding to said acquired information about the targeted amount of daily calcium intake per person specific to the user.

# FIG. 1

# FIG. 2

# FIG. 3

PERSONAL-DATA
REGISTRATION
PROCESS (SET KEY)

S1 — SELECTING PERSONAL NUMBER

S2 — HAS PERSONAL DATA ALREADY BEEN REGISTERED?

YES

S3 — RE-REGISTRATION?

NO

NO

YES

S4 — INPUTTING BIRTH DATE

S5 — INPUTTING GENDER

S6 — INPUTTING BODY HEIGHT

S7 — STORING PERSONAL DATA

END

# FIG. 4

```
        ┌──────────────────────┐
        │   BIOLOGICAL-DATA     │
        │   MEASUREMENT         │
        │   PROCESS (FOOT KEY)  │
        └──────────────────────┘
                   │
                   ▼
      S11        ╱ HAS ╲
            ╱ PERSONAL DATA ╲        NO
           ╱ ALREADY BEEN    ╲──────────────┐            S12
            ╲  REGISTERED?   ╱               │             │
             ╲              ╱                ▼   ┌─────────────────────┐
                  │                          │   │ DISPLAYING MESSAGE  │
                  │ YES                      │   │ ABOUT ABSENCE OF    │
                  ▼                          │   │ REGISTERED PERSONAL │
  S13   ┌──────────────────┐                 │   │ DATA                │
        │ READING PERSONAL │                 │   └─────────────────────┘
        │ DATA             │                 │
        └──────────────────┘                 │
                  │                          │
                  ▼                          │
  S14   ┌──────────────────┐                 │
        │ MEASURING BODY   │                 │
        │ WEIGHT & IMPEDANCE│                │
        └──────────────────┘                 │
                  │                          │
                  ▼                          │
  S15   ┌──────────────────┐                 │
        │ CALCULATING/     │                 │
        │ STORING BODY-    │                 │
        │ COMPOSITION DATA │                 │
        └──────────────────┘                 │
                  │                          │
                  ▼                          │
  S16   ┌──────────────────┐                 │
        │ ACQUIRING        │                 │
        │ REQUIRED Ca      │                 │
        │ INTAKE AMOUNT    │                 │
        └──────────────────┘                 │
                  │                          │
                  ▼                          │
  S17   ┌──────────────────┐                 │
        │ DISPLAYING LATEST│                 │
        │ BIOLOGICAL DATA  │                 │
        └──────────────────┘                 │
                  │                          │
                  ▼◄─────────────────────────┘
           ┌──────────┐
           │   END    │
           └──────────┘
```

# FIG. 5A

161A

| WOMAN | | AGE | | | | | | |
|---|---|---|---|---|---|---|---|---|
| BODY HEIGHT | ESTIMATED BONE MASS | 20'S | 30'S | 40'S | 50'S | 60'S | 70'S | 80'S |
| 145 cm | AVERAGE VALUE OR MORE | 600 | 600 | 650 | 650 | 650 | 650 | 650 |
| | LOW | 650 | 650 | 700 | 700 | 700 | 700 | 700 |
| 150 cm | AVERAGE VALUE OR MORE | 600 | 600 | 650 | 650 | 650 | 650 | 650 |
| | LOW | 650 | 650 | 700 | 700 | 700 | 700 | 700 |
| 155 cm | AVERAGE VALUE OR MORE | 650 | 650 | 650 | 700 | 700 | 700 | 700 |
| | LOW | 700 | 700 | 700 | 750 | 750 | 750 | 750 |
| 160 cm | AVERAGE VALUE OR MORE | 650 | 650 | 650 | 700 | 700 | 700 | 700 |
| | LOW | 700 | 700 | 700 | 750 | 750 | 750 | 750 |
| 165 cm | AVERAGE VALUE OR MORE | 700 | 700 | 700 | 750 | 750 | 750 | 750 |
| | LOW | 750 | 750 | 750 | 800 | 800 | 800 | 800 |
| 170 cm | AVERAGE VALUE OR MORE | 700 | 700 | 700 | 750 | 750 | 750 | 750 |
| | LOW | 750 | 750 | 750 | 800 | 800 | 800 | 800 |
| 175 cm | AVERAGE VALUE OR MORE | 700 | 700 | 700 | 750 | 750 | 750 | 750 |
| | LOW | 750 | 750 | 750 | 800 | 800 | 800 | 800 |

(UNIT OF REQUIRED CALCIUM INTAKE AMOUNT: mg)

# FIG. 5B

161B

| | BODY WEIGHT | LESS THAN 60 kg | 60 kg TO LESS THAN 75 kg | GREATER THAN 75 kg |
|---|---|---|---|---|
| MAN | ESTIMATED BONE MASS AVERAGE VALUE | 2.5 kg | 2.9 kg | 3.2 kg |
| WOMAN | BODY WEIGHT | LESS THAN 45 kg | 45 kg TO LESS THAN 60 kg | GREATER THAN 60 kg |
| | ESTIMATED BONE MASS AVERAGE VALUE | 1.8 kg | 2.2 kg | 2.5 kg |

# FIG. 6A

162A

| BODY MASS INDEX | LARGE | E | F |
|---|---|---|---|
| | AVERAGE | C | D |
| | SMALL | A | B |
| | | LOW | AVERAGE VALUE OR MORE |
| | | BONE MASS | |

# FIG. 6B

162B

| WOMAN | SEGMENT A | | MAN | SEGMENT D |
|---|---|---|---|---|
| AGE GROUP | REQUIRED Ca INTAKE AMOUNT | | AGE GROUP | REQUIRED Ca INTAKE AMOUNT |
| 20'S | 700 mg | | 20'S | 650 mg |
| 30'S | 700 mg | | 30'S | 650 mg |
| 40'S | 700 mg | ........ | 40'S | 650 mg |
| 50'S | 750 mg | | 50'S | 650 mg |
| 60'S | 750 mg | | 60'S | 600 mg |
| 70'S | 750 mg | | 70'S | 600 mg |
| 80'S | 750 mg | | 80'S | 600 mg |
| 90'S | 750 mg | | 90'S | 600 mg |

# FIG. 7

163

| WOMAN | BODY-MASS INDEX (BMI) | | |
|---|---|---|---|
| AGE GROUP | LESS THAN 18.5 (THIN) | 18.5 TO LESS THAN 25 (STANDARD) | GREATER THAN 25 (FAT) |
| 20'S | 1.8 kg | 2.2 kg | 2.5 kg |
| 30'S | 1.8 kg | 2.2 kg | 2.5 kg |
| 40'S | 1.8 kg | 2.2 kg | 2.5 kg |
| 50'S | 1.6 kg | 2.0 kg | 2.2 kg |
| 60'S | 1.6 kg | 2.0 kg | 2.2 kg |
| 70'S | 1.5 kg | 1.8 kg | 2.0kg |
| 80'S | 1.4kg | 1.7 kg | 1.9 kg |
| 90'S | 1.4 kg | 1.7 kg | 1.9 kg |

# FIG. 8

164

| BONE-MASS DIFFERENCE | EXERCISE PERIOD (INITIAL TARGET) | ADVISORY COMMENT | | |
|---|---|---|---|---|
| | | EXERCISE LEVEL | | |
| | | BASAL METABOLIC RATE LOW | BASAL METABOLIC RATE STANDARD | BASAL METABOLIC RATE HIGH |
| −0.1 kg | 3 MONTHS | | TRY TO WALK 10000 STEPS/DAY | |
| −0.2 kg | 6 MONTHS | | | |
| −0.3 kg | 6 MONTHS | WALK WITH A GOAL OF 5000 STEPS/DAY | | |
| −0.4 kg | ONE YEAR | | | |

19

# FIG. 9

```
                        5
┌──────────┐      ⌒⌒⌒      ┌──────────────┐
│ BODY     │ ◄──────────► │ BODY FAT     │
│ WEIGHT   │              │ PERCENTAGE   │
│ 52.1 kg  │              │ 29.3 %       │
└──────────┘              └──────────────┘
```

BODY FAT PERCENTAGE 29.3 %

ESTIMATED BONE MASS 2.1 kg

MUSCLE MASS 34.8 kg

BASAL METABOLIC RATE 1094 kcal/DAY

REQUIRED Ca INTAKE AMOUNT 600 mg/DAY

WALK WITH A GOAL OF 5000 STEPS/DAY FOR INITIAL 3 MONTHS

3.0 CUPS OF MILK

91 91 92 92 93 93 94 94 95 95 95

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5049050 A
- JP 2835662 B
- JP 2005080976 A
- JP 2004344518 A
- JP 2002172099 A